# EUROPEAN PATENT APPLICATION

(11) **EP 1 125 905 A1**
(43) Date of publication of application: **22.08.2001**
(21) Application number: 00200536.1
(22) Date of filing: 16.02.2000
(51) Int. Cl.: C07B 61/00, C07K 1/04, G01N 33/68

(54) **Segment synthesis**

(71) Applicant: PEPSCAN SYSTEMS B.V., 8219 PH Lelystad (NL)
(72) Inventor: Puijk, Wouter, Cornelis, 8243 VZ Lelystad (NL); Slootstra, Jelle, Wouter, 8232 AJ Lelystad (NL); Van Dijk, Evert, 8355 CG Giethoorn (NL)
(74) Representative: Ottevangers, Sietse Ulbe

(57) **Abstract**

The invention relates to the field of molecular recognition or detection of discontinuous or conformational binding sites or epitopes corresponding to a binding molecule, in particular in relation to protein-protein protein-nucleic acid, nucleic acid-nucleic acid or biomolecule-ligand interactions. The invention provides a synthetic molecular library allowing testing for, identification, characterisation or detection of a discontinuous binding site capable of interacting with a binding molecule, said library having been provided with a plurality of molecules, each molecule of said molecules comprising at least one first segment linked to a second segment, each segment having the capacity of being a potential single part of a discontinuous binding site.

## Description

The invention relates to the field of molecular recognition or detection of discontinuous or conformational binding sites or epitopes corresponding to or interacting with a binding molecule, in particular in relation to protein-protein or protein-ligand interactions.

Interactions between binding molecules, which in general are biomolecules, and their corresponding ligands, are central to life. Cells often bear or contain receptor molecules that interact or bind with a hormone, a peptide, a drug, an antigen, an effector molecule or with another receptor molecule; enzymes bind with their substrate; antibody molecules bind with an antigen, nucleic acid with protein, and so on. By "interact or bind" it is meant that the binding molecule and ligand approach each other within the range of molecular forces, and may influence each others properties. This approach takes the binding molecule and its ligand through various stages of molecular recognition comprising increasing degrees of intimacy and mutual effect: they bind. Binding molecules have this binding ability because they comprise distinct binding sites allowing for the recognition of the ligand in question. The ligand, in turn, has a corresponding binding site, and only when the two binding sites can interact by -- essentially spatial -- complementarity, the two molecules can bind. Needless to say that, molecules having three dimensions, binding sites are of a three dimensional nature, often one or more surface projections or protuberances of one binding site correspond to one or more pockets or depressions in the other, a three-dimensional lock-and-key arrangement, sometimes in an induced-fit variety.
Sometimes, such a protuberance comprises a single loop of the molecule in question, and it is only this protuberance that essentially forms the binding site. In that case one often terms these binding sites as comprising a linear or continuous binding site, wherein a mere linear part of the molecule in question is in essence responsible for the binding interaction. This terminology in widely used to describe for example antibody-antigen reactions wherein the antigen comprises part of a protein sequence, a linear peptide. One than often speaks about a linear or continuous epitope, whereby the binding site (epitope) of the antigenic molecule is formed by a loop of consecutively bound amino acids. However, similar continuous binding sites (herein epitope and binding site are use interchangeably) can be found with receptor-antigen interactions (such as with a T-cell *receptor*), with receptor-ligand interactions such as with hormone receptors and agonists or antagonists thereof, with receptor-cytokine interactions or with for example enzyme-substrate or receptor-drug interactions, whereby a linear part of the molecule is recognised as the binding site, and so on.

More often, however, such a protuberance or protuberances and depressions comprise various, distinct parts of the molecule in question, and it are the combined parts that essentially form the binding site. Commonly, one names such a binding site comprising distinct parts of the molecule in question a discontinuous or conformational binding site or epitope. For example, binding sites laying on proteins having not only a primary structure (the amino acid sequence of the protein molecule), but also secondary and tertiary structure (the folding of the molecule into alpha-helices or beta-sheets and its overall shape), and sometimes even quaternary structure (the interaction with other protein molecules) may comprise in their essential protuberances or depressions amino acids or short peptide sequences that lay far apart in the primary structure but are folded closely together in the binding site.

Due to the central role binding molecules and their ligands play in life, there is an ever expanding interest in testing for or identification of the nature or characteristics of the binding site. Not only is one interested in the exact nature of the particular interaction between binding molecule and ligand in question, for example in order to replace or supplement binding molecules or ligands when needed; one is also interested in knowing approximating characteristics of the interaction, in order to find or design analogues, agonists, antagonists or other compounds mimicking a binding site or ligand involved.
Versatile and rapid methods to test for or identify continuous epitopes or binding sites are known. Most, if not all nucleic acid detection techniques, and molecular libraries using these, entail hybridisation of an essentially continuous nucleic acid stretch with a complementary nucleic acid strand, be it DNA, RNA or PNA. Little attention has been paid to methods allowing rapid and straightforward identification of discontinuous binding sites of an essentially nucleic acid nature. Although plenty of such sites exist, think only of the lack of understanding surrounding ribosomal binding sites where ribosomal proteins bind to tRNA, of regulatory sites in promotor sequences, of interactions between polymerases and replicases between DNA and RNA, and so on, no molecular libraries exist that provide easy access to such sites.
An early work in the peptide field is WO 84/03564, related to a method of detecting or determining antigenically active amino acid sequences or peptides in a protein. This work, providing the so-called Pepscan technology, whereby a plurality of different peptides is synthesised by linking with a peptide bond a first amino acid to a second, and so on, and on a second position in the test format yet another first amino acid is linked to a second, and so on, after which the synthesised peptides are each tested with the binding molecule in question, allows the determination of every continuous antigenic determinant or continuous epitope of importance in a protein or peptide sequence. Pepscan technology taken in a broad sense also provides for the testing for or identification of (albeit linear) peptides essentially identical with, analogous to or mimicking binding sites or ligands of a various nature (mimitopes, Geyssen at al, Mol. Immunol. 23:709-715, 1986).
Pepscan technology allows identification of linear peptide sequences interacting with receptor molecules, enzymes, antibodies, and so on, in a rapid and straightforward fashion, allowing testing a great many peptides for their reactivity with the binding molecule in question with relatively little effort. The order of magnitude of testing capability having been developed with Pepscan technology (e.g. also due to miniaturisation of test formats, see for example WO 93/09872) furthermore allows at random testing of a multiplicity of peptides, leading to automated combinatorial chemistry formats, wherein a great many of binding molecules are being tested in a (if so desired at random) pattern for their reactivity with a molecular library of synthetic peptides representing potential continuous binding sites or ligands, allowing the rapid detection of particularly relevant molecules out of tens of thousands of combinations of molecules tested.
However, for the testing of discontinuous or conformational binding sites to a binding molecule, no formats similar to or as versatile as Pepscan technology exist. Attempts to identify discontinuous epitopes by Pepscan technology are cumbersome. It does in general not suffice to merely extend synthesis of the test peptides by linking more amino acids to the existing peptide, and hoping that some of the thus formed longer peptides will fold in such a way that at least two distinct parts are presented in a discontinuous fashion and are recognised by a binding molecule. Than there is no way of finding out in a rapid and straightforward fashion that the binding is indeed through a discontinuous binding site, it might be that just a longer single loop is responsible for the binding.

Some additional possibilities are provided by testing synthetic peptide sequences that have been designed to comprise two previously identified parts of a binding site, each part in essence being linear and being part of a larger linear peptide. Early work herein was done by Atassi and Zablocki (J. Biol. Chem 252:8784, 1977) who describe that spatially or conformationally contiguous surface residues (which are otherwise distant in sequence) of an antigenic site of egg white lysozyme were linked by peptide bonds into a single peptide which does not exist in lysozyme but attempts to simulate a surface region of it. However, their technique, called surface simulation synthesis, requires the detailed knowledge of the three-dimensional structure of the protein under study and a full chemical identification of the residues constituting the binding site at beforehand, as well as their accurate conformational spacing and directional requirements.
In the same fashion, Dimarchi et al (Science 232:339-641, 1986) describe a 38 to 40 amino acid long synthetic peptide consisting of two previously identified separate peptidyl regions of a virus coat protein. The peptide was synthesised using common peptide synthesis technology (Merrifield et al., Biochemistry 21, 5020, 1982) by adding subsequent amino acids with a peptide bond to an ever growing peptide resulting in a peptide wherein the two peptidyl regions were connected by a diproline spacer presumably functioning as indication of a secondary structural turn, thereby thus providing a two-part epitope or binding site.
However, it is clear that when one already at beforehand has to know the sequence of the (in this case only) two relevant parts, in order to provide the desired discontinuous binding site, it excludes the feasibility to provide (desirably in a random fashion) a whole array of merely potential discontinuous binding sites for large scale testing. Furthermore, a major drawback of these strategies is that again only linear epitopes or dominant binding regions of discontinuous eptitopes can be mimicked adequately. For the complete synthesis of a discontinuous binding site, all the contributing parts have to be arranged in the proper comformation to achieve high-affinity binding, therefore, single parts of discontinuous binding sites have to be linked.
Fifteen years after Dimarchi, Reineke et al (Nature Biotechnology, 17:271-275, 1999) provided a synthetic mimic of a discontinuous binding site on a cytokine and a method to find such a discontinuous binding site that allowed for some flexibility and somewhat larger scale of testing, wherein positionally addressable peptide collections derived from two separate regions of the cytokine were displayed on continuous cellulose membranes, and substituted in the process to find the best binding peptide. After selection of the "best reactors" from each region, these were combined to give rise to another synthetic peptide collection (comprising peptides named duotopes) that again underwent several rounds of substitutions.
Reineke et al hereby provide synthesis of peptide chains comprising duotopes, however, again selected after previous identification of putative constituting parts with Pepscan technology, thereby still not allowing testing discontinuous binding sites in a rapid and straight forward fashion. However, as indicated before, protein domains or small molecules that mimic binding sites are playing an increasing role in drug discovery, diagnostics and biotechnology. The search for particular molecules that bind to a binding site and mimic, or antagonise the action of a natural ligand has been initiated in many laboratories. As indicated before, attempts to find such structures in synthetic molecular libraries often fail because of the essentially discontinuous nature and spatial complementarity of most binding sites. Thus, for the many more cases where the binding site may essentially be discontinuous improved means and methods to identify these sites are needed, and in particular means and methods are needed that allow testing for discontinuous binding sites whereby said parts need not necessarily first be selected by previous identification as a putative or even only tentative constituting part of the desired discontinuous binding site, but bear only the potentiality of being part of that site by being a molecule with more or less distinct features per se.

The invention provides a method for producing a molecular library comprising providing said library with a plurality of molecules wherein said molecules have essentially been produced by segmental linkage, that is by linking (di-, tri, oligo- or multimeric) segments of for example nucleic acids or peptides, instead of by sequentially synthesising said molecules which is done traditionally. The invention thus provides a molecular library that, albeit also suited for detecting or screening for continuous binding sites, is now particularly well suited for detecting or screening for discontinuous binding sites, in particular in relation to binding molecule-ligand interactions such as for example protein-protein, protein-nucleic acid, and nucleic acid-nucleic acid interactions.
Said segments can of course be selected at random from any set of di-, tri,- or oligomeric sequences, such as from di-, tri,- or oligonucleotides, or di-, tri-, or oligopeptides, but sometimes it may be preferred to include at least one specific segment in said segment, specific in a sense that it has been selected from among known segments or distinct parts of biomolecules, such as parts of genes, proteins, enzymes, nucleic acids or unique fragments thereof, proteins involved in up- or downregulation of translation, t-RNAs, SNRP's, antibodies, antigens, receptors, transport proteins, transcription factors or factors involved in up- or downregulation of transcription, promotor sequences such as but not necessarily restricted to the well known TATA-box elements, repressor sites, operator sites and other control elements, polymerases, replicases, in short, from among known segments or distinct parts of binding molecules known or suspected to be involved in binding via a discontinuous binding site. Such known segments or parts thereof may of course be already known as parts constituting a discontinuous binding site, however, previous identification as such is in essence not necessary, since screening for such sites with a molecular library according to the invention allows rapid and straightforward identification of said constituting segments or parts thereof.
Screening such a library can easily be envisioned when the library's molecules differ only in that constituting segments are chosen in an overlapping fashion, whereby a first segment from a distinct biomolecule is linked to a second, and to a third, and to a fourth segment, and a second is linked to a third, and to a fourth, and so on, if so required until all possible segments of said biomolecule have been linked two-by-two (or three-by-three, or even more) together, which allows for a systematic screening of said biomolecule. However, linking in a overlapping fashion is of course not required, random segment links will provide valuable information about binding sites as well.
The invention thus provides a method for producing a molecular library for identification or detection of a binding site capable of interacting with a binding molecule, and thus for the identification of a molecule as a binding molecule, said method comprising providing said library with a plurality of molecules, further comprising generating at least one of said molecules, preferably a greater part, most preferably essentially all of said molecules, by at least linking a first segment to a second segment, for example a segment which comprises a dimer, trimer, oligomer or multimer.
Existing libraries, be it of for example nucleic acid (containing a repetitive back-bone of nucleotides, nucleosides or peptide nucleic acid, or combinations of these) or amino acid (containing a repetitive back-bone of amino acids) nature have in general in common that they comprise oligomeric or multimeric molecules, such as stretches of nucleic acids or amino acids, that have been produced by sequentially linking, in a repetitive fashion, one monomer (e.g. a nucleotide or an amino acid) to another, until a (in essence polymeric) molecule of the desired length has been obtained. Essentially, existing nucleic acid libraries comprise nucleic acids that are synthesised sequentially, by adding one nucleotide or nucleoside at a time to the growing stretch, and existing peptide libraries comprise peptides that are synthesised sequentially, by adding one amino acid at the time to a growing stretch, until the desired length has been reached. With nucleic acids said monomers are essentially selected from a limited set of well known nucleotides, with peptides, said monomers are essentially selected from a well known set of amino acids. Not only naturally occurring monomers are used, synthetic nucleotides, such as peptide nucleic acid (PNA) molecules, or non-naturally occurring amino acids, or even D-amino acids, are routinely used as monomers by which the essentially polymeric molecules are generated or produced, using a method that is essentially conform the sequential synthesis of polymers from monomeric molecules in nature.

The invention provides the recognition that essentially using dimeric or even larger (tri-, oligo-, or multimeric) segments, and thus stepping out of fashion with sequential nucleic acid or protein synthesis as it essentially occurs in nature, offers distinct advantages. It not only provides a faster method to arrive at a molecule composed of various segments, it also provides for fast and efficient shuffling of segments to generate a molecule repertoire for the desired library. The invention for example provides a method wherein synthesis is started with a monomer to which a second segment comprising a dimer, such as a dinucleotide or a dipeptide is added. Herein, a segment comprising a dimer at least consists of a dimer but can also be for example a trimer, or any other multimer, linking monomers of any nature, as required. Of course, once two segments have been linked, further segments can be linked.
In a preferred embodiment, to speed up further synthesis, or to be able to select distinct desired segments, the invention provides a method wherein said first segment also comprises a dimer, and in a yet even more preferred method, further segments comprise dimers as well. In a preferred embodiment, said dimer comprises a dinucleotide or dipeptide, but of course other dimers can be made also. The invention is further explained in the detailed description where several of the examples relate to libraries comprising molecules wherein each of said segments comprises a peptide, such as a tri-, a penta, an octa-, or nonapeptide; it is however also provided by the invention to use segments of a varied nature, e.g. wherein one comprises a nucleic acid and another comprises a peptide, to better mimic binding sites that are for example found on nucleic acid-protein complexes.
In a preferred embodiment, as for example shown in the examples, the invention provides a method wherein said first segment is linked by a thioether bond to said second segment, however, the invention is of course not limited thereto. Nucleotide/side segments can for example be covalently linked or ligated by splicing enzymes or ligases, or by overlapping a first segment and the second segment with an in essence relatively short nucleotide strand that is partly complementary to both segments.
The invention thus provides a molecular library allowing testing for, identification, characterisation or detection of a continuous or discontinuous binding site capable of interacting with a binding molecule, said library having been provided with a plurality of molecules, each molecule of said molecules preferably comprising at least one first segment linked to a second segment, wherein at least said second segment previously existed as dimer or multimer. Preferably, each segment or part thereof having the capacity of being a potential single part of a discontinuous binding site, preferably wherein each of at least a first and a second segment or part thereof represents a potential single part of a discontinuous binding site. Such a library can for example exist of a synthetic molecular library made by chemical linking of segments.
Preferably, such segments have distinct features, for example by being in essence segments that are, comprise or mimic molecular components of living organisms, such as (combinations of) nucleotides, sugars, lipids, amino acids, nucleic acid molecules (DNA or RNA), peptide nucleic acid molecules (PNA), carbohydrates, fatty acids or fats. Herewith the invention provides synthesis of molecules comprising separate segments potentially representing at least two distinct parts of a discontinuous binding site, said parts not necessarily first being selected after previous identification of potential constituting parts, thereby allowing testing for discontinuous binding sites in a rapid and straight forward fashion.
The invention thus now allows identifying discontinuous binding sites of receptor molecules that interact or bind at that contact site with a hormone, a peptide, a drug, an antigen, an effector molecule or with another receptor molecule, of enzymes that bind with their substrate, of antibody molecules that bind with a binding site on an antigen, nucleic acid that binds with protein, and so on. In a preferred embodiment of the invention, at least one of said segments comprises a peptide, another segment being for example DNA, RNA, PNA, carbohydrate, a fatty acid, an hormone or an organic molecule altogether. In one embodiment of the invention, all segments comprise a peptide, said segments preferably linked by a stable (non-naturally) occurring non-peptide bond or linker. In this way a plurality of different peptides is synthesised by linking a first peptide segment to a second, and so on, and on a second position in the test or library format yet another first peptide segment is linked to a second, and so on, after which the synthesised peptides are each tested with the binding molecule in question, allowing the determination of a discontinuous antigenic determinant or discontinuous epitope of importance in a protein or peptide sequence.
Said peptide segment comprises at least 2 amino acids, and can in principle be as long as desired, e.g. containing a hundred amino acids or even more. In preferred practice, said peptide segment comprises from 3 to 30, preferably from 4 to 20, even more preferably from 5 or 6 to 12 to 15 amino acids, such as 9 or 12 amino acids. Separate segments of course do not necessarily have to be of equal length.
Furthermore, peptide segments to be linked together can be selected at random, or under guidance of (a) known protein or peptide sequence(s). Selection at random provides a random library according to the invention. Selection from known protein or peptide sequences is for example useful when it is desired to find out whether a discontinuous binding site is composed of distinct sites or parts present at distinct proteins or peptides, for example in a protein complex to which a particular binding molecule can bind. Selection of various peptide segments from one known protein or peptide sequence is useful when it is desired to find out whether a discontinuous binding site is composed of distinct sites or parts present at one protein or peptide, for example at a folded protein to which a particular binding molecule can bind. Selection of peptide segments can be done by selecting overlapping peptides from such a known sequence. Overlapping peptides can have for example all but one or two amino acids in common, preferably overlapping in a contiguous fashion, or can overlap with only one or several amino acids. For a quick scan for discontinuous binding sites on a known protein, it is for example useful to select nonapeptide segments from said protein sequence, of which one has for example a 5-amino acid long overlap with another peptide segment. Equally useful, however, is to select tripeptide segments from said sequence having an overlap of only one amino acid, and use three, or even more segments in constructing the putative binding site molecule to which the to be tested binding molecule can bind.
Of course, such selection strategies are equally applicable to segments of a different nature, nucleic acid segments, comprising a certain number of nucleotides, such as 5, 7, 9, and so on, can be selected from known nucleic acid sequences comprising sought after discontinuous binding sites, each segment selected from a certain position in said known nucleic acid sequence, if desired also in a overlapping fashion. Said nucleic acid segment comprises at least 2 nucleotides (be it DNA, RNA or PNA, or functional equivalents thereof), and can in principle be as long as desired, e.g. containing a hundred nucleotides or even more. In preferred practice, said nucleic segment comprises from 3 to 30, preferably from 4 to 20, even more preferably from 5 or 6 to 12 to 15 nucleotides, such as 9 or 12 nucleotides. Separate segments of course do not necessarily have to be of equal length, and, as said before, can even be of a different nature, e.g. peptide with DNA.
Herein a peptide bond is being defined as an amide bond between an alpha-amino group of one amino acid or peptide and an alpha-carboxyl group of another amino acid or peptide. A non-peptide bond comprises any other amide bond or non-amide bonds. The links or bonds can be formed using many combinations of strategies of for example peptide or nucleotide chemistry and selective ligation reactions as known in the art. Ligation chemistry has been published, for instance, by groups of Kent (Ph.E.Dawson et al., Synthesis of Proteins by Native Chemical Ligation, Science 266 (1994) 776-779), Tam (J.P.Tam et al., Peptide Synthesis using Unprotected Peptides through Orthogonal Coupling Methods, Proc. Natl. Acad. Sci. USA 92 (1995) 12485-12489; C.F.Liu et al, Orthogonal Ligation of Unprotected Peptide Segments through Pseudoproline Formation for the Synthesis of HIV-1 Protease Analogs, J.Am.Chem.Soc. 118 (1996) 307-312; L.Zhang & J.P.Tam Thiazolidone Formation as a General and Site-specific Conjugation Method for Synthetic Peptides and Proteins, Analytical Biochemistry 233 (1996) 87-93), and Mutter (G.Tuchscherer & M.Mutter, Protein Design as a Challenge for Peptide Chemists, J.Peptide Science 1 (1995) 3-10; S.E.Cervigni et al, Template-assisted Protein Design: Chimeric TASP by Chemoselective Ligation, Peptides: Chemistry, Structure and Biology, P.T.P Kaumaya & R.S. Hodges eds, Mayflower (1996) 555-557).
Possible strategies for the formation of links as preferably provided by the invention are for example are:
1. Said link with a segment or segments is formed using a homo- or hetero-bifunctional linking agent (S.S.Wong: Chemistry of Protein Conjugation and Cross-Linking, CRC Press Inc, Boca Raton, Florida USA 1991). In this construction a reactive group in one segment is used to react with one part of the bifunctional linking agent, thus facilitating the second part of the linking agent to react with a reactive group from a second segment. For instance, a linker like MBS (m-maleinimidobenzoic acid N-hydroxysuccinimide ester) can be used to react via its active ester (succinimide) with an amino group of one segment and via its maleinimide group with a free thiol group from a second segment. In this strategy preferably no other free amino- or thiol groups should be present in the first segment and preferably no other free thiol groups are present in the second segment. In order to accomplish this, the amino or thiol groups that should be involved in the reaction can be deprotected selectively, for instance, by using a side chain protecting group that can be cleaved by a mild reagent like 1% trifluoroacetic acid, which leaves other side chain protecting groups intact.
2. Said link is formed by introduction of a modified amino acid in the synthesis of one or more segments. Amino acids can be modified, for instance, by introduction of a special group at the side-chain or at the alpha-amino group. A modification at the alpha-amino group leads to an amide or backbone modified peptide (see fort example Gillon et al., Biopolymers, 31:745-750, 1991). For instance, this group can be a maleinimido group at the side chain amino group of lysine. At the end of the peptide synthesis this group will react fast and selective with a thiol group of a second segment. Tam et al. (PNAS 92:12485-12489, 1995) described a synthesis of a peptide with a lysine residue that was modified in the side chain with a protected serine residue. After deprotection and selective oxidation using periodate, the alpha-amino, beta-hydroxy function of the serine was converted into an aldehyde function that could be ligated selectively with another thiol-bearing segment. Also peptide backbone links, via groups attached to the amide groups of the peptide, can be used to link segments (Bitan et al., J. Chem. Soc. Perkin Trans.1:1501-1510, 1997; Bitan and Gilon, Tetrahedon, 51:10513-10522, 1995; Kaljuste and Unden, Int. J. Pept. Prot. Res. 43:505-511, 1994).
3. Yet another way to form said link is to synthesise a segment, such as a peptide, with a modified N-terminus. For instance, an N-terminal alpha-haloacetamido group can be introduced at the end of the synthesis. This group reacts fast and selectively with a second segment, i.e. another peptide, which contains a thiol group. For instance, the first segment is synthesised with an N-terminal bromoacetamide and the second segment with a cysteine. Although most alpha-haloacetamide groups, like chloro-, bromo-, or iodoacetamide, will react with thiol groups, in those cases where speedy assembling is required, the bromoacetamide group is preferred because of its ease of introduction and fast and selective reaction with thiol groups.
Furthermore, the invention provides the possibility to address the link in every position of the first and/or the second or consecutive segment. For instance, for peptide segments sets of peptides are synthesised in which a cysteine or a side-chain modified lysine, both amino acid residues being able to ligate selectively with another segment, shifts from the N-terminal amino acid position one by one to the C-terminal amino acid position. Combinations of these possibilities will again lead to new libraries as provided by the invention.
In a preferred embodiment, the invention provides a library wherein said molecules are positionally or spatially addressable, e.g. in an array fashion, if desired aided by computer directed localisation and/or recognition of a specific molecule or set of molecules within the dimensions (e.g. plane or surface) of the support of the library used. In an array, said molecules are for example addressable by their positions in a grid or matrix.
A preferred embodiment of the invention further allows upscaling of the synthesis concerning the number of constructs on for example a solid support per cm². To facilitate generation of a great many possible constructs, containing for example molecules comprising at least two peptide segments of a protein, many thousands of peptide constructs are made. For instance, when all constructs, in which both segments are for instance 12 amino acids long, are derived from a small protein with a length of 100 amino acid residues are needed, already 89 x 89 = 7921 peptide constructs are made, if the segments are only linked, for instance, via the C-terminus of the first segment and the N-terminus of the second segment using only one type of link. For a protein with a length of 1000 amino acid residues at least 989 x 989 = 978121 constructs are made. For efficient ELISA testing of these numbers of constructs, high construct densities on the solid support are preferred. High densities of constructs on a solid support are provided by the invention, wherein for instance, (a layer of) a first segment with a bromoacetamide group at the N-terminus is synthesised on a surface of, for instance, 1 cm². On this peptide-functionalised surface of the support a set of, for instance, 10, preferably 50, preferably 100, or more second peptide segments containing a free thiol group are spotted or gridded, in a positionally or spatially addressable way, giving, after coupling, so many different peptide constructs. Spotting can, for instance, be done using piezo drop-on-demand technology, or by using miniature solenoid valves. Gridding can, for instance, be done using a set of individual needles that pick up sub-microliter amounts of segment solution from a microtiter plate, containing solutions comprising the second segments. After the linking reaction, subsequent deprotection and extensive washing of the support to remove uncoupled peptide gives at least a peptide construct density as large as 10 to 50, or even 100 to 200, or up to 50 to 1000 spots per cm². This density allows to screen a great many possible peptide constructs of said proteins for binding with an antibody. For example: in a preferred embodiment 20000 to 100.000 constructs are made on 1000 cm², typically the surface is than screened for binding in ELISA with 100 ml of antibody solution, containing 1 - 10 *µ*g of antibody/ml. For example, indirect or direct fluorescence detection allocates antibody binding constructs. Direct fluorescence detection with confocal scanning detection methods for example allows antibody detection on spots generated with droplets peptide-solution in the sub-nanoliter range, making even higher construct densities feasible. Of course, nucleic acid libraries can be made in a similar fashion.
Furthermore, the invention provides a solid support comprising a library according to the invention, said solid support allowing presentation of a potential discontinuous or conformational binding site or epitope to a binding molecule, said solid support having been provided with a plurality of molecules, each molecule of said molecules being a possible representative of said binding site or epitope and for example comprising at least one first peptide or nucleotide covalently linked by a spacer to a second peptide or nucleotide, said spacer comprising at least one non-peptide linkage.
In a preferred embodiment, said solid support comprises at least a spot or dot (e.g. putative binding site or peptide construct) density as large as 10, 20, or 50, or even 100, 200, or up to 500 or even 1000 spots per cm²' preferably wherein said spots or dots are positionally or spatially addressable.
The invention further provides a method to screen for, i.e. test, identify, characterise or detect a discontinuous binding site capable of interacting with a binding molecule, comprising screening a library as provided by the invention with binding molecules, such as there are for instance antibodies, soluble receptors, which contain a Fc-tail or a tag for detection, receptors on cells, biotinylated molecules or fluorescent molecules. Alternative segments could comprise, for instance, carbohydrates, non-natural amino acids, PNA's, DNA's, lipids, molecules containing peptide bond mimetics. In particular, the invention provides a method to screen for a discontinuous binding site capable of interacting with a binding molecule, comprising screening a library according to the invention with at least one binding molecule and detecting binding between a member of said library and said binding molecule. In a preferred embodiment, said binding is detected immunologically, for example by ELISA techniques.
By detecting binding to a specific member of said library, the invention provides said member, a synthetic molecule comprising a discontinuous binding site identifiable or identified or obtainable or obtained by a method according to the invention. Thus the invention provides use of a library according to the invention, use of a solid support according to the invention, or use of a method according to the invention for identifying or obtaining a synthetic molecule comprising a discontinuous binding site or a binding molecule capable of binding therewith. Because now discontinuous binding sites are provided, such a synthetic molecule can advantageously be used in vitro or in vivo for finding a binding molecule, and for effecting and/or affecting binding to a binding molecule, for example to interact or bind with receptor molecules that normally interact or bind with a hormone, a peptide, a drug, an antigen, with an effector molecule, with an agonist, with an antagonist, or with another receptor molecule; with enzymes that normally bind with their substrate; with antibody molecules, with nucleic acid, with protein, in short, with biomolecules. The invention is further explained in the detailed description without limiting the invention.

### Figure legends.

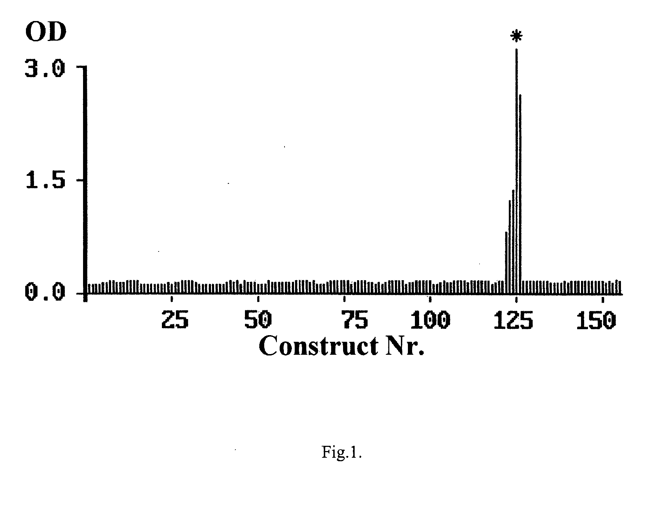

Fig. 1. ELISA results of a library of constructs of a protein, synthesised in 3 *µ*l wells of a 455 wells microtiter plate tested against a protein specific monoclonal antibody. Construct 1: sequence[1-11]-Cys coupled to bromoacetamide-sequence[14-25]; construct 2: sequence[2-12]-Cys coupled to bromoacetamide-sequence[15-26]; and so on.

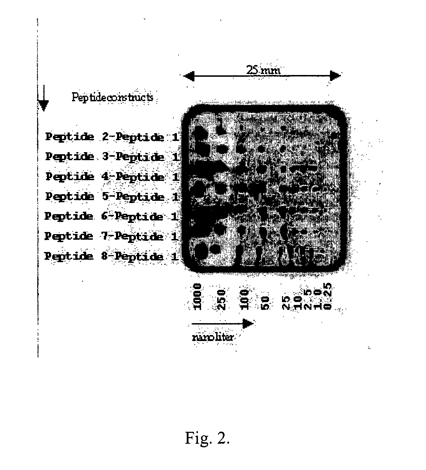

Fig. 2. Microturisation of spots of peptide constructs. Constructs were tested against the same monoclonal antibody as was tested in figure 1. Binding was made visible using indirect fluorescence detection. Peptide 1, sequence [139 - 150] with an N-terminal bromoacetamide, was synthesised on the complete surface. Peptide construct 6 - 1 is the same as construct 125 in figure 1. Peptides 2 up to 8, containing a cysteine residue, were spotted in different volumes ranging from 1 *µ*l to 0.25 nl using piezo drop-on-demand technology.

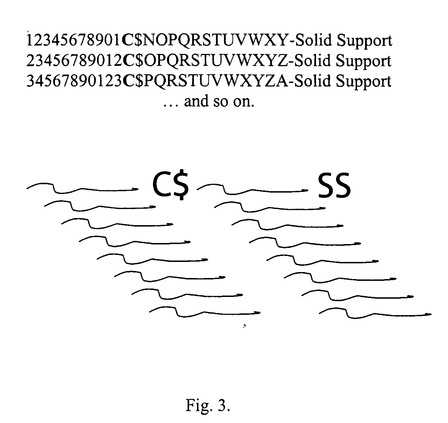

Fig. 3. Schematic presentation of a two segment scan. 12345678901 (segment 2) and NOPQRSTUVWXY (segment 1) represent successive sequences derived from a protein. Both segments are linked via a thioether bridge, formed by reaction of a free thiol function of a C-terminal cysteine residue (C) in segment 2 and a bromoacetamide group ($) at the N-terminus of segment 1. In this scan both sequences can be shifted simultanuously by steps of one amino acid residue through the complete protein sequence to obtain the library. SS = Solid Support.

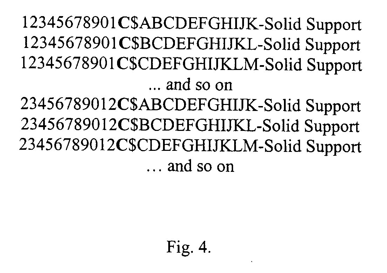

Fig. 4. Schematic presentation of a complete matrix scan. 12345678901 and ABCDEFGHIJK represent sequences derived from a protein. Both sequences are linked as described in fig. 3. In this scan both sequences are shifted independently through the complete protein sequence, generating a library of all possible sequence combinations.

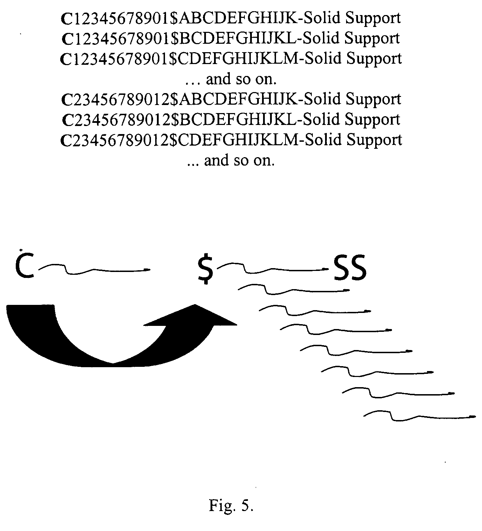

Fig. 5. Schematic presentation of a complete matrix scan. This scan is similar to the scan shown in fig. 4, however, the cysteine residue is positioned at the N-terminus of the second segment, leading to a reversed of both segments.

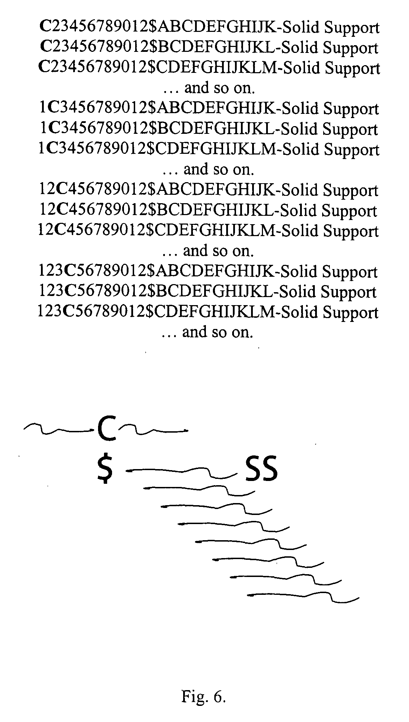

Fig. 6. Schematic presentation of a positional complete matrix scan. This scan is similar to the scans shown in figs. 4 and 5, however, no cysteine residue was added to one of the termini of the second segment, but instead each residue of the second segment sequence was substituted one by one by a cysteine residue.

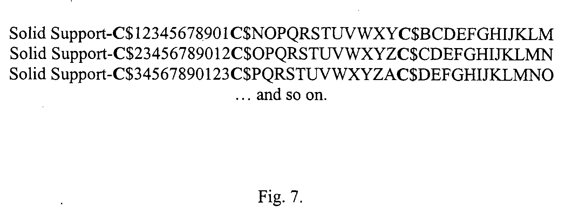

Fig. 7. Schematic presentation of a multi segment scan. 12345678901 (segment 1), NOPQRSTUVWXY (segment 2) and

BCDEFGHIJKLM (segment 3) represent successive sequences derived from a protein. Segments were linked via a thioether bridge, formed by reaction of a free thiol function of a C-terminal cysteine residue (C) in segment 1 and a bromoacetamide group ($) at the N-terminus of segment 2 and so on, as described in example 3. In this scan all sequences are subsequently shifted simultanuously through the complete protein sequence to obtain the complete library.

Fig. 8. Schematic presentation of a scan of interacting proteins 1, 2 and 3. *ABCDEFGHIJK* and ABCDEFGHIJK represent two independent sequences from two different proteins combined in one construct. **A:** a matrix scan of these segments was tested against a complete, labelled third protein. **B:** a matrix scan of segments from HIV proteins and the CCR5 protein was tested against a labelled soluble CD4 protein.

Fig. 9. Schematic presentation of a matrix combi-scan with a complete protein. The constructs are scanned with a another labelled protein in solution.

Fig. 10. Schematic presentation of a DNA/RNA scan. The constructs are scanned with a labelled protein, for example a regulatory protein, or another DNA or PNA strand.

Fig. 11. Schematic presentation of a PNA/DNA test. The upper part shows the principle of a test. A cysteine modified PNA was reacted with a bromoacetylated solid support, the bound PNA was tested against a biotin labelled DNA strand. In an ELISA system the labelled DNA was detected using HRPO labelled streptavidine. The lower part of the figure shows the reactivity in ELISA of the PNA with respectively a complementary DNA strand, a complementary strand with a single mutation, an unrelated DNA strand and a buffer control. SS = solid support.

Fig. 12. Schematic presentation of an intracellular protein scan.

### Detailed description

### SYNTHESIS OF PEPTIDE CONSTRUCTS

A peptide with a N-terminal bromoacetamide group was synthesised at the surface of a solid support containing free amino groups. The peptide still contained the side-chain protecting groups of the amino acid residues. A second peptide containing a cysteine residue, which was deprotected and cleaved from another solid support was reacted with the bromoacetamide peptide on the first solid support. The formed construct was deprotected, but not cleaved from the support, and could be used in ELISA.
A polypropylene or polyethylene support, or of other suitable material, was grafted with, for instance, polyacrylic acid. As an example: a polypropylene support in a 6 % acrylic acid solution in water, containing CuSO₄, was irradiated using gamma radiation at a dose of 12 kGy. The grafted solid support containing carboxylic acid groups was functionalised with amino groups via coupling of t-butyloxycarbonyl-hexamethylenediamine (Boc-HMDA) using dicyclohexylcarbodiimide (DCC) with N-hydroxybenzotriazole (HOBt) and subsequent cleavage of the Boc groups using trifluoroacetic acid.
Standard Fmoc peptide synthesis was used to synthesise peptides on the amino functionalised solid support. After cleavage of the Fmoc group of the last amino acid and washing, bromoacetic acid was coupled using DCC or DCC/HOBt. If only DCC was used the peptide did contain a thiol reactive bromoacetamide group, however, if DCC/HOBt was used to couple bromoacetic acid, the peptide essentially did not contain the bromo group, but another reactive group capable to react efficiently with thiol groups thus forming the same thioether link between the segments.

Coupling/ligation of a second peptide to a peptide synthesised on a solid support:
Peptides were synthesised at polyethylene pins grafted with poly-hydromethylmethacrylate (poly-HEMA). This graft polymer was made by gamma irradiation of polyethylene pins in a 20% HEMA solution in methanol/water 80/20 or 70/30 at a dose of 30-50 kGy. The functionalised support can be used for the synthesis of 1 *µ*mol of peptide/cm² after coupling of β-alanine and an acid labile Fmoc-2,4-dimethoxy-4'-(carboxymethyloxy)-benzhydrylamine (Rink) linker. The peptides were synthesised using standard Fmoc chemistry and the peptide was deprotected and cleaved from the resin using trifluoroacetic acid with scavengers.
The cleaved peptide containing a cysteine residue at a concentration of about 1 mg/ml was reacted with the solid support bound peptide described above in a water/sodium bicarbonate buffer at about pH 7-8, thus forming a partially protected construct of two peptides covalently bound via a thioether and C-terminally bound to the solid support.
The construct described above was deprotected following standard procedures using trifluoroacetic acid/scavenger combinations. The deprotected constructs on the solid support were extensively washed using disrupting buffers, containing sodium dodecylsulphate and β-mercaptoethanol, and ultrasonic cleaning and were used directly in ELISA. Subsequent cleaning in the disrupt buffers allows repeatingly testing against other antibodies in ELISA.

Figure 1 shows an example of the ELISA results of screening a simple library of constructs, consisting of a dodecapeptide segment coupled via its C-terminally added cysteine residue to a N-terminally bromoacetylated second segment, scanning a protein sequence by steps of a single amino acid residue. The bromoacetamide peptide was covalently bound to a functionalised polypropylene/polyacrylic acid solid support in 3 *µ*l wells as described above. The cysteine-containing sequences were synthesised on and cleaved from functionalised polyethylene pins as described above. As shown in figure 1, high binding was observed in ELISA for constructs around position 125, which consists of the segments [125-136] and [139-150], linked via a thioether bond. A conventional linear PEPSCAN of dodecapeptides or 15-peptides did not show any binding in a reaction against the same monoclonal antibody. On a surface of a solid support peptides are synthesized with a bromoacetamide group at the N-terminus as described above. On this peptide functionalized support a second peptide segment containing a free thiol group was spotted using piezo drop-on-demand technology, using a microdosing apparatus and piezo autopipette (Auto Drop-Micropipette AD-K-501) (Microdrop Gesellschaft fur Mikrodosier Systeme GmbH. Alternatively, spotting or gridding was done using miniature solenoid valves (INKX 0502600A; the Ice Company) or hardened precision ground gridding pins (Genomic Solutions, diameters 0.4, 0.6, 0.8 or 1.5 mm). Subsequent deprotection of the construct and extensive washing to remove uncoupled peptide gave dipeptide constructs at the spotted area.
Figure 2 shows binding of the same antibody as was tested in figure 1 with constructs consisting of two peptide segments, generated with different volumes of spotted peptides 2 to 8, ranging from 1 *µ*l - 0.25 nl (x-axis). Within the square the whole surface was covered with peptide 1, which was synthesised directly on this surface, only the spots contain constructs. The y-axis shows different constructs, consisting of peptide 1 with peptide 2 up to 8. Peptides 2 up to 8 are overlapping dodecapeptides, while peptide 1 is sequence [139-150] of the same protein as described in figure 1. Figure 2 shows that peptide constructs generated with peptide solution droplets in the nanoliter-range, bind enough antibody for detection, in this case using indirect fluorescence detection. Spots generated with 0.25 nl - 50 nl are smaller than 1 mm². Thus, peptide construct density can be as large as 100-1000 spots per cm².

### Examples of use

Proteins and peptides can be screened using any type of binding molecule, e.g. biomolecules such as antibodies, soluble receptors, which contain a Fc-tail or a tag for detection, biotinylated molecules or fluorescent molecules. Alternative segments could be, for instance, carbohydrates, non-natural amino acids, PNA's, DNA's, lipids, molecules containing peptide bond mimetics. In the examples $ is used as a symbol for a thioether link formed by reaction of the thiol group of a cysteine residue of one segment with a bromoacetamide group from another segment. This symbol can also be used for other linking chemistries as described, and thus describes links between segments.

### Example-1: 'standard' 24-30-mer scan of linear sequence, containing two segments.

In this example the consecutive sequences of the segments are both shifted one by one residue through the sequence of the protein to be tested as shown in figure 3. The -C$- link between both segments replaces 0 - 2 or more amino acid residues of the native protein sequence. Applications include replacement sets of peptides, in which amino acid residues are replaced systematically by other amino acid residues, deletion sets of peptides, in which amino acid residues are deleted systematically, and combination sets, in which peptides of different lenght ranging from 2 - 24 (here segment 2, 2-40 or more and segment 1, 2-15 or more) amino acid residues are used.

### Example-2a: tail-to-head matrix-scan.

In a complete matrix-scan the N-terminal sequence of, for instance, sequence [1 - 11] of a protein, is linked as a segment with each overlapping peptide sequence of a complete scan of the same protein as shown in figure 4. Next, sequence [2 - 12] is linked with the same set of overlapping sequences and so on. The link can be formed, for instance, by reaction of a cysteine at the C-terminus of the second segment with a bromoacetamide modified N-terminus of the first segment. This means that every combination of, for instance, undecapeptides from the protein sequence is being synthesised on a separate, known, position of the solid support.

### Example-2b: head-to-head matrix-scan.

This is the same scan as the complete matrix scan from example 2a, however, in this scan the cysteine of the second segment is located at its N-terminus, providing a reversed orientation of both segments in the construct as shown in figure 5.

### Example-2c: Positional matrix-scan with cysteine at ALL positions.

This is a scan similar to the complete matrix scans described above, however, in this scan the cysteine is used to substitute the amino acid residues one by one in every position of the second segment as shown in figure 6.

### Example 2d: tail-to-tail or head-to-tail matrix scan.

Tail-to-tail or head-to-tail matrix scans can be obtained if for example a cysteine is incorporated a C-terminal amino acid or a first segment on the solid support. A second segment is added either as N-terminal bromoacetylated peptide or as C-terminal side chain bromoacetylated lysyl peptide.

### Example-3: Multi-segment scan.

In this example a thiol fuction is introduced on an amino-functionalised solid support. This can be made by a direct reaction of the amino groups with, for instance, iminothiolane, or by coupling of Fmoc-Cys(Trt)-OH, followed by Fmoc cleavage using piperidine, acetylation, and trityl deprotection using TFA/scavenger mixtures. This thiol-functionalised solid support can be reacted with, for instance, a bromoacetamide-peptide, containing a protected cysteine residue. After coupling of the first peptide, the cysteine can be deprotected, using, for instance, a TFA/scavenger mixture. The formed free thiol group can be used to couple a second bromoacetamide-peptide, again containing a protected cysteine. This procedure can be repeated to make multi-segment constructs. Several types of scans, as described in the other examples, can be used in combination with this multi-segment scan. In fig. 7 an example is shown for a multi-segment scan.

### Example-4: Matrix combi-scan, interaction between three (or more) proteins

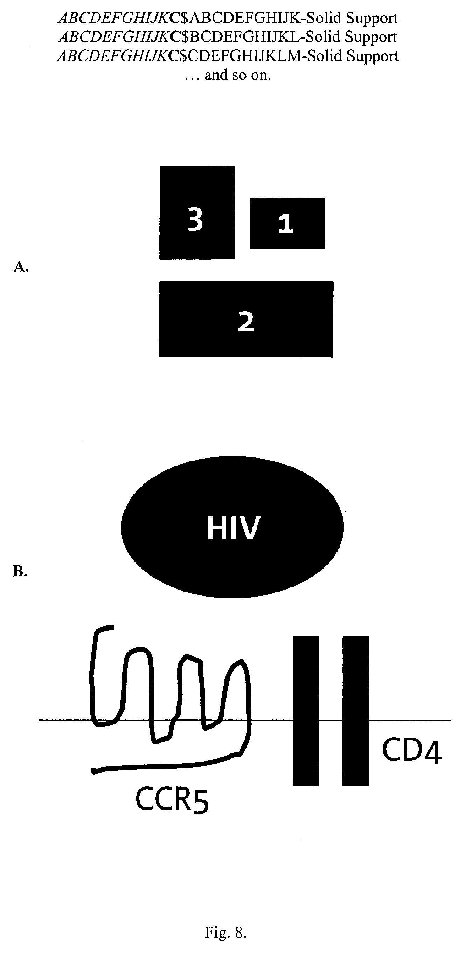

In a matrix combi-scan, a matrix scan from two different proteins is tested against a labelled soluble protein. Figure 8 shows two examples. In the first example (fig. 8A) soluble protein 1 (growth hormone, GH) was tested against a combined matrix scan of protein 2 (GH-receptor-1) and protein-3 (GH-receptor-2). In the second example a part of soluble protein 1 (CD-4) was tested against a combined matrix scan of protein 2 (HIV) and protein 3 (chemokine-receptor CCR4).

Examples 1 to 4 describe methods using peptide segments and screening with proteins. These constructs can also be screened against non-proteins. Also non-peptide segments can be used. Below, examples of whole proteins in combination with peptides (example 5), or peptides/proteins in combination with non-peptide/proteins, or non-peptide/protein with non-peptide/protein (example 6, DNA/RNA/PNA) are shown.

### Example-5: Matrix combi-scan, interaction between three (or more) proteins.

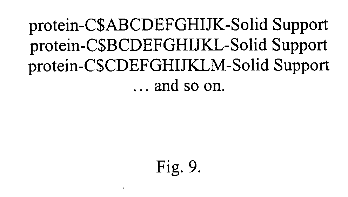

This example is similar to example 4. The difference is that the segment 2 sequences, derived from one protein (*ABCDEFGHIJKC* etc.) are replaced by a complete protein, which contains an added thiol group for coupling (see fig 9).

### Example-6: scans of linear DNA/PNA/peptide with DNA/PNA/peptide

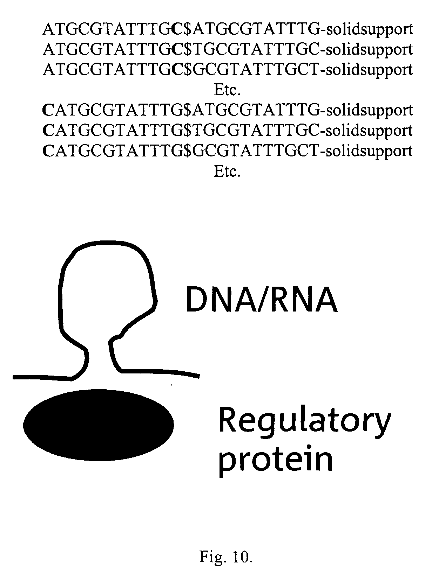

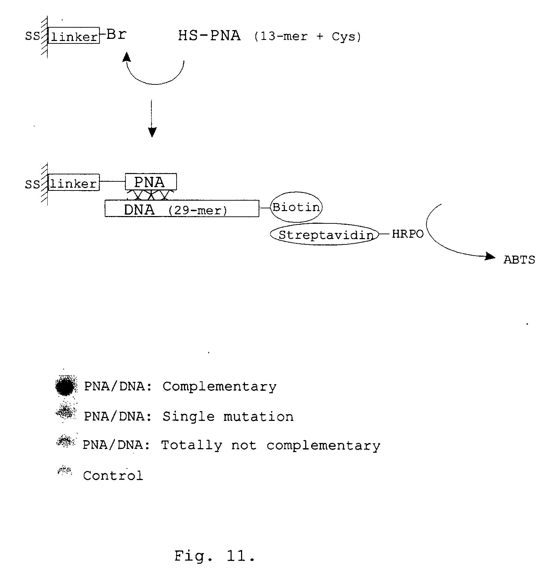

This example is similar to that of examples 1 to 5 with the difference that one or more other non-peptide segments are used (DNA, RNA or a peptide nucleic acid (PNA) instead of a peptide segment). The nucleotide segments or PNA's are modified with groups that enable linking of the segments as in examples 1 to 5. Screening is performed with labelled DNA strands, peptides or proteins (see fig 10 or 11). As alternative labelled DNA or PNA strands can also be tested against peptide construct described in examples 1 to 5.

In addition to scanning interaction regions of proteins and non-proteins (DNA/RNA) in ELISA, chip or blot format it is also possible to use the -C$- coupling in *in vitro* bioassays. Firstly, it is possible to use soluble constructs as explained in example-3 as potential (ant)agonists for membrane bound receptors. Secondly, it is possible to use membrane-transporting proteins such as transportan or penetratin to get any of the above mentioned combinations of peptides or peptides with PNA or peptides with (small) proteins into the cell. Below two examples are shown.

### Example-7a: Intracellulair protein scan coupled to membrane-penetrating Transportan.

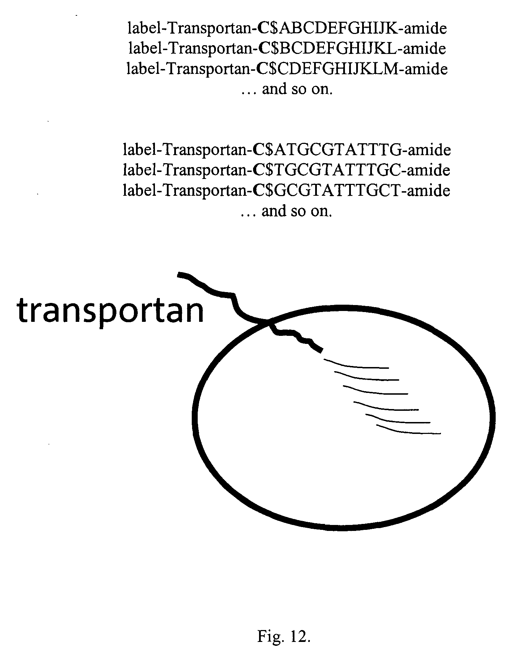

An intracellulair protein, like a kinase, can be scanned using overlapping peptides on a solid support, containing a C-terminal cleavable linker. The peptides were synthesised with a N-terminal bromoacetamide group. Next, a membrane penetrating transportan peptide, containing a label and a thiol group was coupled with the sequences. These constructs were selectively cleaved from the solid support and tested in a bioassay. Labels that can be used are, for instance, biotine or fluorescent labels (figure 12).

### Example-7b: Intracellulair RNA, DNA or PNA-scan coupled to membrane-penetrating Transportan.

To identify PNA/DNA sequences that can be used to block expression of a particular gene a long linear scan, coupled to membrane penetrating peptide, can be tested in an in vitro bio-assay.

## Claims

1. A method for producing a molecular library for identification or detection of a binding site comprising providing said library with a plurality of molecules, further comprising generating at least one of said molecules by at least linking a first segment to a second segment.

2. A method according to claim 1 wherein said first or second segment comprises at least a dimer.

3. A method according to claim 1 or 2 wherein said dimer comprises a dinucleotide or dipeptide.

4. A method according to anyone of claims 1 to 3 wherein each of said segments comprise a peptide.

5. A method according to anyone of claims 1 to 4 wherein said first segment is linked by a thioether bond to said second segment.

6. A method according to anyone of claims 1 to 5 wherein each of at least a first and/or a second segment or part thereof represents a potential part of a discontinuous binding site.

7. A library comprising a plurality of molecules comprising at least a first and a second segment obtainable by a method according to anyone of claims 1 to 6.

8. A library according to claim 7 wherein said molecules are positionally or spatially addressable.

9. A library according to claim 7 or 8 wherein each of at least a first and/or a second segment or part thereof represents a potential part of a discontinuous binding site.

10. A solid support comprising a library according to anyone of claims 7 to 9.

11. A method to screen for a binding site capable of interacting with a binding molecule, comprising screening a library according to anyone of claims 7 to 9 with at least one potential binding molecule and detecting binding between a member of said library and said potential binding molecule.

12. A method according to claim 11 wherein said binding site is a discontinuous binding site.

13. A synthetic molecule comprising a binding site identifiable or obtainable by a method according to claim 11 or 12.

14. A binding molecule comprising a binding site identifiable or obtainable by a method according to claim 11 or 12.

15. A molecule according to claim 13 or 14 wherein said binding site comprises a discontinuous binding site.

16. Use of a library according to anyone of claims 7 to 9, a solid support according to claim 10, or a method according to claim 11 or 12 for identifying or obtaining a synthetic molecule comprising a binding site.

17. Use of a library according to anyone of claims 7 to 9, a solid support according to claim 10, or a method according to claim 11 or 12 for identifying or obtaining a binding molecule capable of binding to a binding site.

18. Use of a molecule according to claim 13, 14 or 15 for interfering with or effecting binding to a binding molecule.
